# EUROPEAN PATENT APPLICATION

(11) **EP 4 756 819 A1**
(43) Date of publication of application: **10.06.2026**
(21) Application number: 23948577.4
(22) Date of filing: 14.08.2023
(51) Int. Cl.: G16H 20/00, G16H 10/65, G16H 40/00

(54) **DIGITAL THERAPY METHOD USING IDENTIFICATION MEDIUM, AND APPARATUS USING METHOD**

(30) Priority: 04.08.2023 KR 20230102209
(71) Applicant: WELT Corp., Ltd, Seocho-gu Seoul 06628 (KR)
(72) Inventor: KANG, Seong Ji, Seoul 06366 (KR); ROH, Hye Kang, Seoul 05507 (KR); KIM, Joo Young, Seoul 04014 (KR); LEE, Do Hyun, Yongin-si Gyeonggi-do 16826 (KR); JEONG, Hwa Young, Incheon 21451 (KR)
(74) Representative: AWA Sweden AB
(86) International application number: PCT/KR2023/012018
(87) International publication number: WO 2025/033582

(57) **Abstract**

The present invention relates to a digital therapy method using an identification medium, and an apparatus using the method. The digital therapy method using an identification medium may comprise steps in which a user device: receives a digital therapy application on the basis of the identification medium; and transmits user digital therapy data related to a digital therapy service to a digital therapy server on the basis of the digital therapy application.

## Description

### BACKGROUND

### 1. Field of the Invention

The present disclosure relates to a method of digital treatment using an identification medium and an apparatus using the method. More particularly, the present disclosure relates to a method of digital treatment using an identification medium, such as a physical card, for providing a treatment service based on digital therapeutics, and an apparatus using the method.

### 2. Discussion of Related Art

With the development of various smart technologies, data related to personal daily activities is being recorded, and personal lives may be managed more efficiently based on the recorded data. Among the various smart technologies, health-related data logging has gained attention as interest in health has increased. Many users are already generating and utilizing various health-related data, such as exercise, diet, and sleep of users, through user devices, such as smartphones and wearable devices. This represents a shift from the past in which health-related data was generated and managed only by medical institutions to a situation in which users have begun to generate and manage their own health-related data through user devices, such as smartphones or wearable devices.

Health-related data logging is often performed through wearable devices. A wearable device is a user device that is carried or attached to a user's body. With advancements of the Internet of Things (IoT) and the like, wearable devices are being widely used to collect health-related data. Wearable devices may collect information about a user's physical changes and environmental data surrounding the user, and provide advice required for the user's health based on the collected data.

Health-related data of a user may include user biomarkers, and research is being conducted on a method of adaptively performing medical prescriptions for users based on health-related data of users.

The related art is disclosed in Korean Registered Patent No. 10-2425479.

### SUMMARY OF THE INVENTION

The present disclosure aims to solve all of the problems described above.

Furthermore, an aim of the present disclosure is to install a digital treatment app on a user apparatus based on an identification medium and provide a digital treatment service based on the digital treatment app to a user.

Furthermore, an aim of the present disclosure is to utilize a user's digital treatment data between different digital treatment apps and utilize the user's digital treatment data generated from the digital treatment app in a medical service apparatus.

### [Means for solving the Problems]

A representative configuration of the present invention for achieving the above objects is as follows.

According to an aspect of the present invention, there is provided a method of digital treatment using an identification medium, comprises receiving, by a user apparatus, a digital treatment app based on an identification medium; and transmitting a user's digital treatment data related to a digital treatment service to a digital treatment server based on the digital treatment app.

Meanwhile, the identification medium includes a physical card, and the digital treatment app is installed on the user apparatus based on a user identification code corresponding to the physical card.

Further, the user's digital treatment data is transmitted between different digital treatment apps based on a standardized format.

According to another aspect of the present invention, there is provided an apparatus for a user apparatus for receiving a digital treatment using an identification medium, wherein the user apparatus is configured to receive a digital treatment app based on an identification medium, and transmit a user's digital treatment data related to a digital treatment service to a digital treatment server based on the digital treatment app.

Meanwhile, the identification medium includes a physical card, and the digital treatment app is installed on the user apparatus based on a user identification code corresponding to the physical card.

Further, the user's digital treatment data is transmitted between different digital treatment apps based on a standardized format.

### [BRIEF DESCRIPTION OF DRAWINGS]

FIG. 1 is a conceptual diagram illustrating a digital treatment system according to an embodiment of the present disclosure.
FIG. 2 is a conceptual diagram illustrating a digital therapeutic provision platform according to an embodiment of the present disclosure.
FIG. 3 is a conceptual diagram illustrating a method of providing a digital treatment service according to an embodiment of the present disclosure.
FIG. 4 is a conceptual diagram illustrating transfer of a user's treatment data between digital treatment apps according to an embodiment of the present disclosure.
FIG. 5 is a conceptual diagram illustrating a method of providing a user's digital treatment data to a medical service server according to an embodiment of the present disclosure.
FIG. 6 is a conceptual diagram illustrating an operation of a digital treatment service platform for controlling a conflict between digital treatment apps according to an embodiment of the present disclosure.

### DETAILED DESCRIPTION OF EXEMPLARY EMBODIMENTS

The detailed description of the present invention will be made with reference to the accompanying drawings showing examples of specific embodiments of the present invention. These embodiments will be described in detail such that the present invention can be performed by those skilled in the art. It should be understood that various embodiments of the present invention are different but are not necessarily mutually exclusive. For example, a specific shape, structure, and characteristic of an embodiment described herein may be implemented in another embodiment without departing from the scope and spirit of the present invention. In addition, it should be understood that a position or arrangement of each component in each disclosed embodiment may be changed without departing from the scope and spirit of the present invention. Accordingly, there is no intent to limit the present invention to the detailed description to be described below. The scope of the present invention is defined by the appended claims and encompasses all equivalents that fall within the scope of the appended claims. Like reference numerals refer to the same or like elements throughout the description of the figures.

Hereinafter, in order to enable those skilled in the art to practice the present invention, exemplary embodiments of the present invention will be described in detail with reference to the accompanying drawings.

Hereinafter, embodiments of the present invention will be described for the sake of convenience of description with an apparatus for phenotype-based digital treatment for treating insomnia by way of an example. However, the embodiments of the present invention may employ phenotype-based digital treatment algorithms for various other diseases as well as insomnia, and such embodiments may also be included within the scope of the present invention.

Hereinafter, "identification medium" may be broadly interpreted to refer to various types of recognizable objects, such as a physical card, a digital card, and a quick response (QR) code. Such embodiments are also within the scope of the present disclosure.

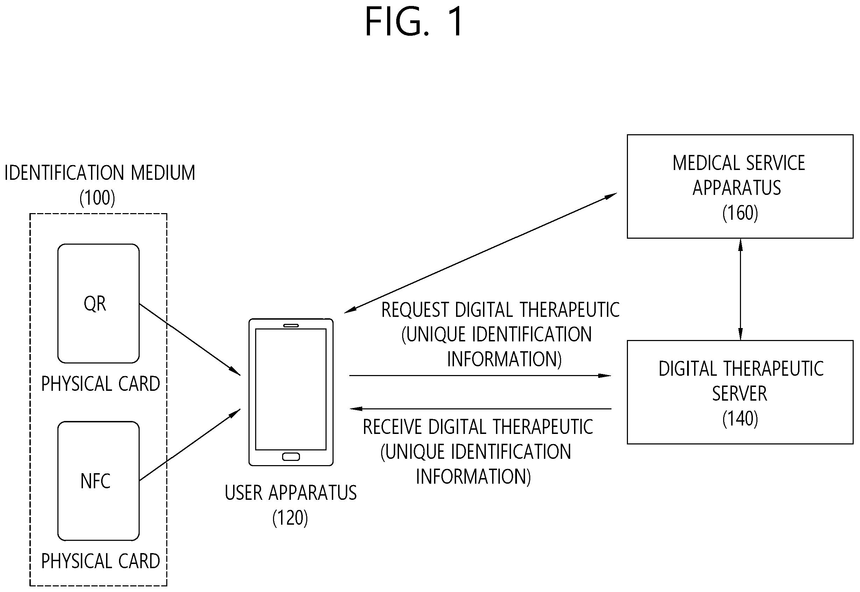

FIG. 1 is a conceptual diagram illustrating a digital treatment system according to an embodiment of the present disclosure.

Referring to FIG. 1, a method of digital treatment using an identification medium such as a physical card is disclosed.

Referring to FIG. 1, the digital treatment system may include an identification medium 100, a user apparatus 120, a digital treatment server 140, and a medical service apparatus 160.

The identification medium 100 may be a medium for installing a digital treatment app on the user apparatus, such as a physical card. For example, when there is a QR code on the physical card and the QR code is read by the user apparatus 120, the digital treatment application (digital treatment app) may be downloaded to the user apparatus 120. When the digital treatment app is downloaded, a user identification code for identifying a user may be assigned.

The user apparatus 120 may install the digital treatment app based on the identification medium. After the installation of the digital treatment app, the user's digital treatment data generated based on the digital treatment app may be transmitted to the digital treatment server 140 based on the user identification code.

A plurality of digital treatment apps may be installed on the user apparatus 120, and a user may perform digital treatment by selecting one or more applications from the plurality of digital treatment apps. A digital treatment app management platform capable of managing the plurality of digital treatment apps may be provided on the user apparatus 120 and manage the plurality of digital treatment apps.

The digital treatment server 140 may be implemented to manage and process the user's digital treatment data and the user identification code, and to provide digital treatment services. The digital treatment server 140 may be implemented as a server corresponding to a specific digital treatment app or to provide services corresponding to the plurality of digital treatment apps.

The digital treatment server 140 may provide a digital treatment service to a user through the digital treatment app based on the user's digital treatment data and the user identification code.

The medical service apparatus 160 may be an apparatus for providing direct medical services on an offline/online basis. For example, the medical service apparatus 160 may be an apparatus installed in an offline hospital. When a user visits a medical institution, the user's digital treatment data may be provided to the medical service apparatus 160. For example, a user visiting a hospital for insomnia treatment may select a digital treatment app related to insomnia and the hospital's medical service apparatus 160, thereby transmitting the user's existing digital treatment data to the medical service apparatus 160. For example, when a user permits the transmission of the user's digital treatment data related to the insomnia treatment, the digital treatment data may be transmitted from the insomnia-related digital treatment server 140 to the medical service apparatus 160.

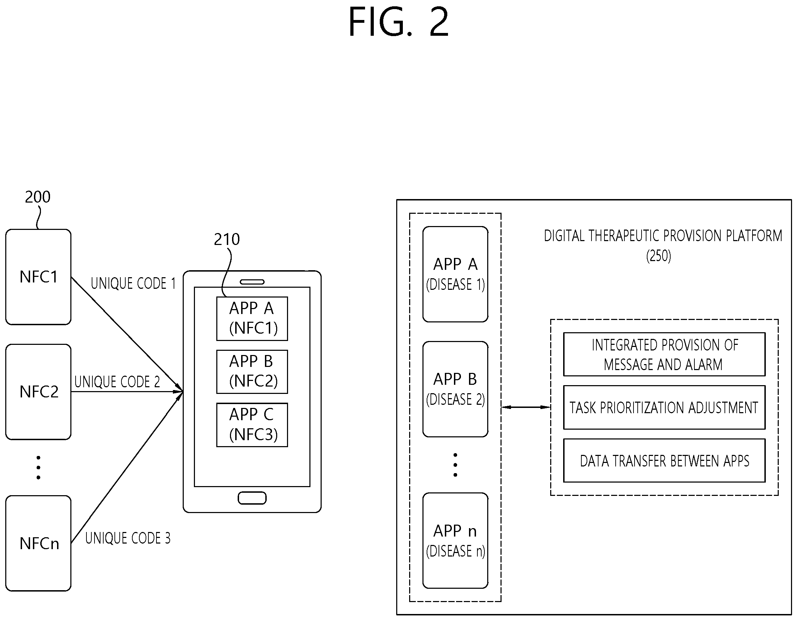

FIG. 2 is a conceptual diagram illustrating a digital therapeutic provision platform according to an embodiment of the present disclosure.

Referring to FIG. 2, a method of providing digital treatment services based on multiple digital therapeutics is disclosed.

Referring to FIG. 2, a digital therapeutic provision platform 250 is installed on a user apparatus and may manage multiple digital therapeutics 200.

A user may download the plurality of digital treatment apps 210 corresponding to the multiple digital therapeutics 200 and receive digital treatment services through the user apparatus. The digital therapeutic provision platform 250 may be implemented to manage the plurality of digital treatment apps 210. A user may select one of the plurality of digital treatment apps 210 through the digital therapeutic provision platform 250 and receive digital treatment services. Messages or alarms transmitted from plurality of digital treatment apps 210 may be provided to a user through the digital therapeutic provision platform 250.

For example, it may be assumed that digital treatment app A, digital treatment app B, and digital treatment app C are running on the digital therapeutic provision platform 250. Alarms (such as an alarm requesting specific information input, a medication administration alarm, and lifestyle-related alarms) from each of digital treatment app A, digital treatment app B, and digital treatment app C may be provided through the digital therapeutic provision platform 250.

Furthermore, according to an embodiment of the present disclosure, when a conflict occurs between the plurality of digital treatment apps 210, the digital therapeutic provision platform 250 may adjust operations of the plurality of digital treatment apps 210 based on priorities among the plurality of digital treatment apps 210. For example, when a medication conflict that requires administration at a specific time occurs or a conflict occurs between operations that should be performed at a specific time, it is possible to determine a task to be preferentially performed in consideration of priorities, and to perform adjustment on other tasks.

In addition, according to an embodiment of the present disclosure, data transfer between the digital treatment apps 210 having the same or similar roles may be performed through the digital therapeutic provision platform 250. For example, it may be assumed that there are a digital treatment app X and a digital treatment app Y for treating insomnia. A user may use the digital treatment app X for treating the insomnia and then change the digital treatment app X to the digital treatment app Y. In this case, among the user's digital treatment data generated while using the digital treatment app X, the user's digital treatment data that is available through the digital treatment app Y may be provided to a digital treatment server corresponding to the digital treatment app Y. Through this method, a user may receive more accurate digital treatment services by directly utilizing the user's existing digital treatment data even when using another digital treatment app.

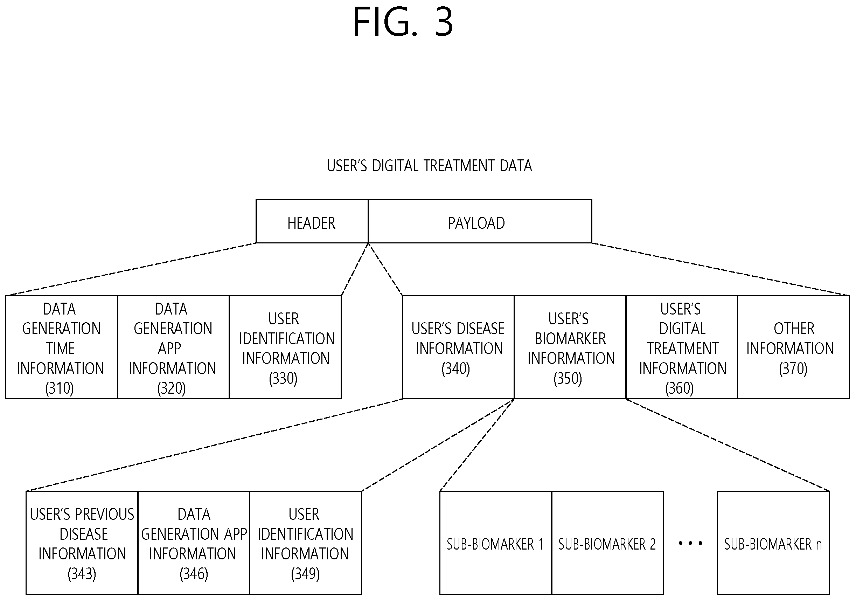

FIG. 3 is a conceptual diagram illustrating a method of providing a digital treatment service according to an embodiment of the present disclosure.

Referring to FIG. 3, a method of sharing a user's digital treatment data between digital treatment apps is disclosed.

Referring to FIG. 3, the user's digital treatment data is defined based on a standardized format and may be transmitted between different digital treatment apps and utilized.

The user's digital treatment data may include a header and a payload.

The header may include data generation time information 310, data generation app information 320, and user identification information 330.

The data generation time information 310 may include information regarding the time at which the user's digital treatment data was generated.

The data generation app information 320 may include information regarding an app from which the user's digital treatment data is generated. For the digital treatment app, a specific app identification code may be assigned, and the app identification code allows identification of information regarding an application from which the information is generated.

The user identification information 330 may include identification information regarding the user whose digital treatment data is generated. For the digital treatment app, the user identification information may be managed in an integrated manner. For example, offline identification information, such as a resident registration number, may be utilized as the user identification information to identify users undergoing digital treatment.

The payload may include the user's disease information 340, the user's biomarker information 350, the user's digital treatment information 360, and other information 370.

The user's disease information 340 may include disease code information regarding the user's disease. The user's disease information 340 may be composed of three sub-information items: the user's previous disease information 343, the user's current disease information 346, and the user's predicted disease information 349.

The user's previous disease information 343 may include the information regarding the user's disease that has occurred previously. The user's current disease information 346 may include the information regarding the user's current disease. The user's predicted disease information 349 may include the information regarding the user's predicted disease.

The user's biomarker information 350 may include biomarker information for determining a user status. The user's biomarker information 350 may include a plurality of pieces of sub-biomarker information. The plurality of pieces of sub-biomarker information may be defined for biomarkers used for digital treatment. Each of the plurality of pieces of sub-biomarker information may include biomarker generation time, biomarker information, and biomarker generation device information. The user's biomarkers that cannot be obtained among the user's biomarker information 350 may be displayed as a null status. The user's biomarker information 350 may be additionally defined when there is a new biomarker for digital treatment.

A standardized biomarker data type may be defined for each individual biomarker, and sub-biomarker information may be defined according to the standard biomarker data type. The plurality of pieces of sub-biomarker information may be defined to be grouped and arranged at adjacent positions as the biomarkers have the same or similar roles or the same or similar effects.

The user's digital treatment information 360 may include a record of the user's digital treatment based on digital therapeutics. For example, information regarding medications, treatment algorithms, etc., may be included in the user's digital treatment information 360.

The user's digital treatment information 360 may additionally include associated biomarker information that indicates which user biomarkers the prescribed medications and treatment algorithms were determined based on.

Other information 370 may include information that can be individually utilized for digital treatment in the digital treatment app.

By defining the header and payload in the above-described manner, the digital treatment may be performed using the user's digital treatment information 360 that has been generated previously in different digital treatment apps.

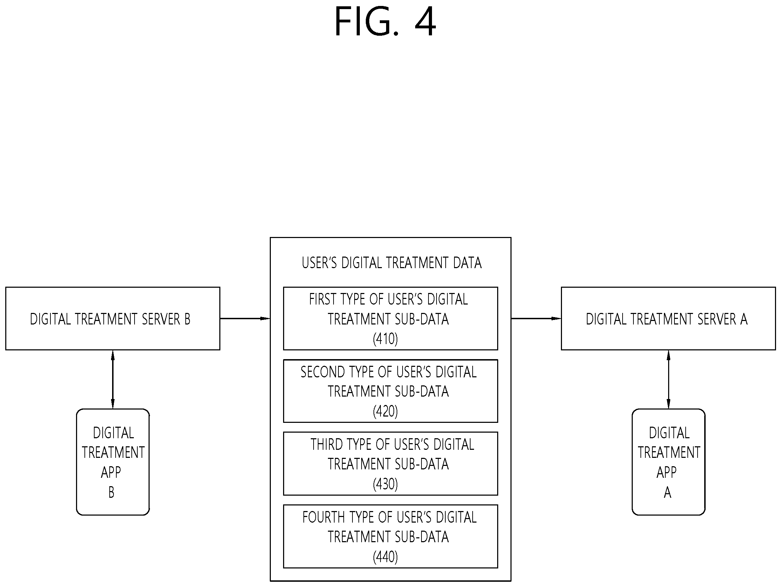

FIG. 4 is a conceptual diagram illustrating transfer of a user's treatment data between digital treatment apps according to an embodiment of the present disclosure.

Referring to FIG. 4, a method of transmitting a user's digital treatment data between digital treatment apps is disclosed.

Referring to FIG. 4, to transmit the user's digital treatment data between the digital treatment apps, only some of the user's digital treatment data may be selectively transmitted.

The user's digital treatment information and other information included in the payload are information related to the treatment of the digital treatment app. Therefore, among the digital treatment apps, the transmission of the user's digital treatment information and other information may be allowed only between the digital treatment apps for which mutual transmission is permitted.

The user's digital treatment data may be classified into four types of the user's digital treatment sub-data, depending on whether the user's digital treatment data can be transmitted to other digital treatment apps.

The first type of the user's digital treatment sub-data 410 is the user's digital treatment sub-data that can be transmitted without user consent. The second type of the user's digital treatment sub-data 420 is the user's digital treatment sub-data that can be transmitted based on user consent. The third type of the user's digital treatment sub-data 430 is the user's digital treatment sub-data that can be transmitted based on settings between the digital treatment apps, regardless of user consent. The fourth type of the user's digital treatment sub-data 440 is the user's digital treatment sub-data that cannot be transmitted.

Through this setting, the transmission of the user's digital treatment data may be performed according to options.

For example, a digital treatment server A, corresponding to the digital treatment app A newly installed on a user apparatus, may request the user's digital treatment data from a digital treatment server B, corresponding to another digital treatment app B, based on user consent information, related digital treatment app information, etc. A digital treatment server B may transmit the user's transmittable digital treatment data to the digital treatment server A by confirming the user consent and the related digital treatment app.

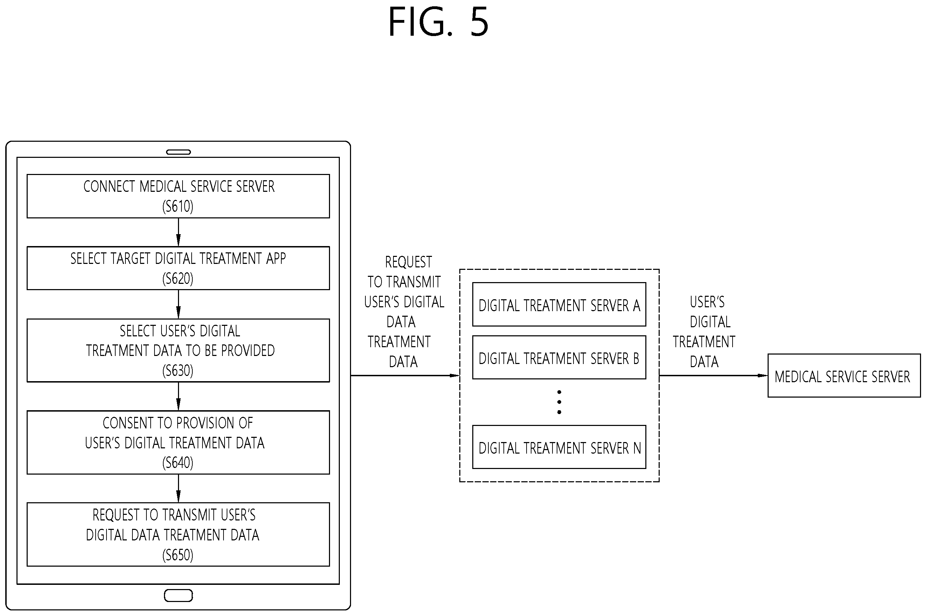

FIG. 5 is a conceptual diagram illustrating a method of providing a user's digital treatment data to a medical service server according to an embodiment of the present disclosure.

Referring to FIG. 5, a method of transmitting user's digital treatment data to a medical service institution to receive medical services is disclosed.

Referring to FIG. 5, when a user visits a medical institution to receive medical services or receives medical services online, the user's digital treatment data that is accumulated previously is transmitted to the medical service server of the medical service institution, thereby allowing offline or online treatment to be performed based on this data.

To provide the user's digital treatment data, the digital treatment server may be connected to the medical service server via the user apparatus (operation S610).

The medical service server may transmit the information regarding the related digital treatment app to the user apparatus based on the information regarding the digital treatment app installed on the user apparatus.

Depending on the type of medical service required, the medical service server may select a digital treatment app among the digital treatment apps installed on the user apparatus as a related digital treatment app for receiving the user's digital treatment data.

The user may select, from the related digital treatment apps, the target digital treatment app to which the user's digital treatment data will be provided through the user apparatus (operation S620).

In this case, a user may exclude a digital treatment app from which the user does not wish to transmit the user's digital treatment data from the related digital treatment apps.

The user's digital treatment data to be provided from the target digital treatment app may be selected through the user apparatus (operation S630).

Among the user's digital treatment data generated by the target digital treatment app, any of the user's digital treatment data that the user does not wish to provide to the medical service server is excluded, and only the user's remaining digital treatment data may be transmitted to the medical service server.

The provision of the user's digital treatment data by the target digital treatment app may be consented to through the user apparatus (operation S640).

After the consent is obtained through the user apparatus, the user apparatus may request the transmission of the user's digital treatment data via the digital treatment server (operation S650).

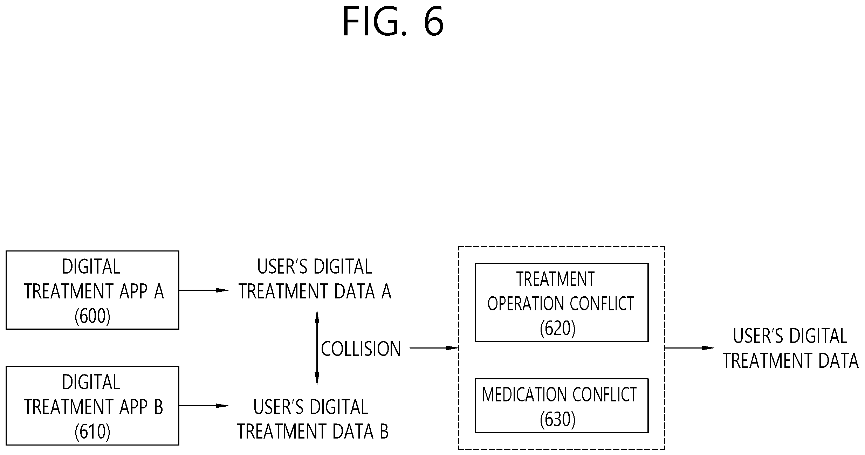

FIG. 6 is a conceptual diagram illustrating an operation of a digital treatment service platform for controlling a conflict between digital treatment apps according to an embodiment of the present disclosure.

Referring to FIG. 6, a method of preventing a conflict between the operations of digital treatment apps in a digital treatment service platform is disclosed.

Referring to FIG. 6, when a conflict occurs between a user's digital treatment data A generated by a digital treatment app A 600 and the user's digital treatment data B generated by a digital treatment app B 610, the user's digital treatment data of the digital treatment app may be generated by considering a conflict between digital therapeutics.

The digital treatment service platform may determine the user's digital treatment data of the digital treatment app by considering the conflict between the user's digital treatment data of the plurality of digital treatment apps installed on the user apparatus.

The digital treatment app A 600 may provide a meal algorithm for eating disorders as the user's digital treatment data, based on a digital treatment module. The digital treatment app B 610 may also provide a sleep algorithm for treating insomnia as the user's digital treatment data, based on the digital treatment module. For example, when a recommended sleep time from the digital treatment app B 610 overlaps with a meal time from the digital treatment app A 600, a conflict may occur between solutions generated by the digital therapeutics, since sleeping and eating cannot be performed simultaneously.

As another example, a medication conflict may occur between administered medications A suggested by the digital treatment app A for disease A and administered medications B suggested by the digital treatment app B for disease B.

Therefore, when the plurality of digital treatment apps are operating on the user apparatus, the digital treatment service platform may determine the possibility of conflict between the user's digital treatment data from each of the plurality of digital treatment apps.

The conflicts in the user's digital treatment data of the digital treatment app may be classified as a treatment operation conflict 620, a medication conflict 630, etc., depending on the types of the conflicts.

The treatment operation conflict 620 may occur when a conflict occurs between operations that should be performed based on the user's digital treatment data from a plurality of digital treatment apps provided at a specific time. The digital treatment service platform may determine the possibility of treatment operation conflicts occurring at the same time by analyzing the operation between the plurality of pieces of the user's digital treatment data that are provided on an hourly basis.

A medication conflict 630 may occur when a conflict arises between medications that should be taken based on the user's digital treatment data from a plurality of digital treatment apps provided at a specific time. The digital treatment service platform is capable of determining the possibility of medication conflicts by considering a medication intake time and a time during which the medication exerts its effects that are included in the plurality of pieces of the user's digital treatment data provided to the user on an hourly basis.

The digital treatment service platform may determine the possibility of the treatment operation conflict 620 and medication conflict 630, and when there is the possibility of a treatment operation conflict 620 or medication conflict 630, may adjust the user's digital treatment data.

When there is the treatment operation conflict 620, the digital treatment service platform may determine which operations will be performed preferentially based on the priority of each action. For example, it is possible to determine relative priorities between sleep time in insomnia and meal time in eating disorders. The relative priority may be determined by considering the severity of the user's disease and the treatment effect of the corresponding operation on the user's disease. In other words, the priorities are determined differently for each user, thereby allowing for different digital treatment data to be provided in the event of the treatment operation conflict.

Based on the determined priorities, actions with higher priority may be performed preferentially when conflicts between operations occur. Hereinafter, the operations that are not performed may either not be performed or their execution time may be adjusted to a different time based on the characteristics of the operation.

When the medication conflict is possible, the digital treatment service platform may determine whether conflict avoidance is possible through dosage adjustments. When the dosage adjustment is feasible, the user's digital treatment data with the adjusted dosage may be provided through the digital treatment app. When conflicts may not be avoided through individual medication dosage adjustments, the priority of each medication may be considered to determine which medication should be administered first. For example, the relative priority of medications may be determined by considering the severity of the disease and the treatment effect of each medication on the disease.

To eliminate such conflicting effects between medications, for a medication that is assigned a lower priority, the medication intake time may be postponed and adjusted accordingly.

According to an embodiment of the present disclosure, the medication effects and side effects resulting from conflicts between multiple medications may be determined as follows.

To consider the medication effects and side effects of a combination of the multiple medications, a combined embedding value for the medication combination may be determined based on the individual embedding values of each medication.

The individual embedding values of the medications may be positioned on a two-dimensional embedding plane based on the effects and side effects of the individual medication components, as medication component 1 {effect, side effect}, medication component 2 {effect, side effect}, and medication component n {effect, side effect}.

According to an embodiment of the present disclosure, a combined embedding value for predicting conflicts considering the effects and side effects of combination medications is generated based on a combination of individual embedding values, and may be determined by considering the individual embedding values, their density, and the relationships between the individual embedding values.

Operation 1: To determine the combined embedding value, the individual embedding values of each of the multiple medications may be positioned on the embedding plane. In this case, when a specific individual embedding value among the individual embedding values has an effect that is less than or equal to a threshold effect or has a characteristic in which a side effect is not strong and is less than or equal to a threshold side effect, the specific individual embedding value may be excluded.

Operation 2: By considering the relationships among the individual embedding values, the individual embedding values of each of the multiple medications may be adjusted based on whether the change in effects (for example, a decrease or an enhancement of effects) or the change in side effects (for example, a decrease or an enhancement of side effects) occurs due to a combination of the medications. For example, by considering the case where the effects are enhanced or the side effects are reduced, the individual embedding values representing the degree of an effect and the degree of a side effect may be adjusted to adjust the position on the embedding plane.

Operation 3: The combined embedding value may form clusters that reflect the effects and side effects by clustering the individual embedding values. A dense cluster having a density equal to or greater than a threshold density may be determined based on the density of the individual embedding values within the cluster. The cluster may be performed for each effect or each side effect. That is, the clustering may be configured to be performed among identical or similar effects and identical or similar side effects, such that the dense cluster is determined for each effect and for each side effect.

The combined embedding value may be finally determined based on the density of the individual embedding values within the dense cluster, and a prediction of an increase or decrease in a specific side effect or an increase or decrease in a specific effect when a combination of multiple medications is taken may be performed. For example, when a density of a dense cluster corresponding to a specific effect is equal to or greater than a threshold density, the increase in the effect may be predicted, and when a density of a dense cluster corresponding to a specific side effect is equal to or greater than a threshold density, the increase in the side effect may be predicted.

According to the present disclosure, the digital treatment app can be installed on the user apparatus based on the identification medium and the digital treatment service based on the digital treatment app can be provided to the user.

Furthermore, according to the present disclosure, the user's digital treatment data can be utilized between different digital treatment apps and the user's digital treatment data generated from the digital treatment app can be utilized in the medical service apparatus.

The embodiments of the present invention described above may be implemented in the form of program instructions that can be executed through various computer units and recorded on computer readable media. The computer readable media may include program instructions, data files, data structures, or combinations thereof. The program instructions recorded on the computer readable media may be specially designed and prepared for the embodiments of the present invention or may be available instructions well known to those skilled in the field of computer software. Examples of the computer readable media include magnetic media such as a hard disk, a floppy disk, and a magnetic tape, optical media such as a compact disc read only memory (CD-ROM) and a digital video disc (DVD), magneto-optical media such as a floptical disk, and a hardware device, such as a ROM, a RAM, or a flash memory, that is specially made to store and execute the program instructions. Examples of the program instruction include machine code generated by a compiler and high-level language code that can be executed in a computer using an interpreter and the like. The hardware device may be configured as at least one software module in order to perform operations of embodiments of the present invention and vice versa.

While the present invention has been described with reference to specific details such as detailed components, specific embodiments and drawings, these are only examples to facilitate overall understanding of the present invention and the present invention is not limited thereto. It will be understood by those skilled in the art that various modifications and alterations may be made.

Therefore, the spirit and scope of the present invention are defined not by the detailed description of the present invention but by the appended claims, and encompass all modifications and equivalents that fall within the scope of the appended claims.

## Claims

1. A method of digital treatment using an identification medium, comprising:
receiving, by a user apparatus, a digital treatment app based on an identification medium; and
transmitting a user's digital treatment data related to a digital treatment service to a digital treatment server based on the digital treatment app.

2. The method of claim 1, wherein the identification medium includes a physical card, and
the digital treatment app is installed on the user apparatus based on a user identification code corresponding to the physical card.

3. The method of claim 2, wherein the user's digital treatment data is transmitted between different digital treatment apps based on a standardized format.

4. A user apparatus for receiving digital treatment using an identification medium,
receive a digital treatment app based on an identification medium, and
transmit a user's digital treatment data related to a digital treatment service to a digital treatment server based on the digital treatment app.

5. The user apparatus of claim 4, wherein the identification medium includes a physical card, and
the digital treatment app is installed on the user apparatus based on a user identification code corresponding to the physical card.

6. The user apparatus of claim 5, wherein the user's digital treatment data is transmitted between different digital treatment apps based on a standardized format.
